# EUROPÄISCHE PATENTSCHRIFT

(11) **EP 3 849 343 B1**
(45) Veröffentlichungstag und Bekanntmachung des Hinweises auf die Patenterteilung: **12.07.2023**
(21) Anmeldenummer: 18769342.9
(22) Anmeldetag: 12.09.2018
(51) Int. Cl.: A23L 33/10, A61K 36/906, A61K 36/9066, A61K 31/095, A61K 31/724, A61K 36/02, A61K 36/67, A23L 33/105, A23L 33/135, A23L 33/15, A23L 33/22, A61K 31/12, A61K 31/375, A61K 31/4525, A61K 36/03, A61K 36/9068

(54) **ZUSAMMENSETZUNG, NAHRUNGSERGÄNZUNGSMITTEL, ZUSAMMENSETZUNG ALS NAHRUNGSERGÄNZUNGSMITTEL FÜR KINDER UND VERFAHREN ZUR HERSTELLUNG**
COMPOSITION, FOOD SUPPLEMENT, COMPOSITION ACTING AS A FOOD SUPPLEMENT FOR CHILDREN, AND PRODUCTION METHOD
COMPOSITION, COMPLÉMENT ALIMENTAIRE, COMPOSITION EN TANT QUE COMPLÉMENT ALIMENTAIRE POUR ENFANTS ET PROCÉDÉ DE FABRICATION

(43) Veröffentlichungstag der Anmeldung: 21.07.2021
(73) Patentinhaber: PM-International AG, 5445 Schengen (LU)
(72) Erfinder: SORG, Rolf, 5444 Schengen (LU); KÜHNE, Tobias, 54497 Morbach (DE); LAUINGER, Christian, 76275 Ettlingen (DE); MESSER, Wilhelm, 67098 Bad Dürkheim (DE)
(74) Vertreter: Meissner Bolte Partnerschaft mbB
(86) Internationale Anmeldenummer: PCT/EP2018/074588
(87) Internationale Veröffentlichungsnummer: WO 2020/052745

(56) Entgegenhaltungen:
- EP-A1- 3 345 623
- EP-A2- 2 415 358
- DATABASE GNPD [Online] MINTEL; 22. Dezember 2017 (2017-12-22), anonymous: "Multi-Vitamin Dietary Supplement", XP055580069, gefunden im www.gnpd.com Database accession no. 5329837
- DATABASE GNPD [Online] MINTEL; 15. August 2018 (2018-08-15), anonymous: "KidGreenz Natural Tropical Fruit Flavour Children's Chewable Tablets", XP055580058, gefunden im www.gnpd.com Database accession no. 5894201
- BHUSHAN MUNJAL ET AL: "Comparative oral bioavailability advantage from curcumin formulations", DRUG DELIVERY AND TRANSLATIONAL RESEARCH, Bd. 1, Nr. 4, 11. Mai 2011 (2011-05-11), Seiten 322-331, XP055579919, Germany ISSN: 2190-393X, DOI: 10.1007/s13346-011-0033-3
- SAHDEO PRASAD ET AL: "Recent Developments in Delivery, Bioavailability, Absorption and Metabolism of Curcumin: the Golden Pigment from Golden Spice", CANCER RESEARCH AND TREATMENT, Bd. 46, Nr. 1, 1. Januar 2014 (2014-01-01) , Seiten 2-18, XP055427457, KR ISSN: 1598-2998, DOI: 10.4143/crt.2014.46.1.2

## Beschreibung

Die Erfindung betrifft eine Zusammensetzung, ein Nahrungsergänzungsmittel, eine Zusammensetzung als Nahrungsergänzungsmittel für Kinder und ein Verfahren zur Herstellung einer Zusammensetzung, eines Nahrungsergänzungsmittels oder einer Zusammensetzung als Nahrungsergänzungsmittel für Kinder. Nahrungsergänzungsmittel werden beispielweise in der EP 3 345 623 A1 und der EP 2 415 358 A2 beschrieben.

Der Einsatz von Nahrungsergänzungsmitteln, beispielsweise im Sport oder zur Förderung der Gesundheit, ist bereits bekannt. Nahrungsergänzungsmittel sind auch dazu geeignet, eine unausgewogene Ernährung zu ergänzen. Im Gegensatz zu konventionellen Lebensmitteln und Getränken stellen Nahrungsergänzungsmittel üblicherweise ein Konzentrat von Nahrungsergänzungsstoffen oder sonstigen Stoffen mit ernährungsspezifischer, physiologischer Wirkung dar. Sie werden in dosierter Form, insbesondere in Form von Kapseln, Pastillen, Tabletten, Brausetabletten, Pulverbeuteln, Flüssigampullen und Flaschen mit Tropfeinsätzen, zur Aufnahme in abgemessenen Kleinmengen vertrieben. Oft enthält ein Nahrungsergänzungsmittel mehr als einen bestimmten Stoff, zum Beispiel mehrere Vitamine und/oder Mineralstoffe in einem vorbestimmten Verhältnis. Manche Nahrungsergänzungsmittel enthalten auch bestimmte Antioxidantien. Da bei der Herstellung der meisten Nahrungsergänzungsmittel synthetische Verbindungen eingesetzt werden, enthalten die Präparate im Ergebnis oft eine überschaubare Anzahl an Inhaltsstoffen. Wenig spricht gegen die Verwendung synthetischer Inhaltsstoffe, allerdings wird dabei nicht die ganze Kraft der Natur ausgenutzt. Insbesondere wird die große Anzahl verschiedener pflanzlicher Sekundärmetabolite und Polyphenole nicht in dem Ausmaß eingesetzt, wie sie die Natur zur Verfügung stellt. Dabei weisen pflanzliche Sekundärmetabolite wie die Polyphenole erwiesenermaßen zahlreiche gesundheitsfördernde Eigenschaften mit synergistischen Wirkungen auf.

Letztlich werden durch Nahrungsergänzungsmittel die Stoffe bereitgestellt, die der Konsument in einer entsprechenden Situation benötigt, beispielsweise der Sportler beim Ausüben eines Ausdauersportes. Letztlich ist es jedoch nicht nur entscheidend, welche Stoffe bereitgestellt werden, sondern auch wie diese bereitgestellt werden und inwiefern es gelingt, die Nahrungsergänzungsstoffe dem Körper und letztlich der einzelnen Zelle des menschlichen Körpers zur Verfügung zu stellen. Diese Bioverfügbarkeit wird insbesondere bei der Gabe konzentrierter Nahrungsergänzungsmittel als verbesserungswürdig angesehen.

Es ist daher eine Aufgabe der vorliegenden Erfindung, eine Zusammensetzung, ein Nahrungsergänzungsmittel und eine Zusammensetzung als Nahrungsergänzungsmittel für Kinder anzugeben, bei welchen eine oder mehrere Komponenten gemeinsam mit einer Mischung verschiedenster pflanzlicher Sekundärmetabolite in synergistischer Art und Weise ihre gesundheitsfördernde Wirkung entfalten können und gleichzeitig die Bioverfügbarkeit der Komponenten verbessert ist. Ferner soll ein Verfahren bereitgestellt werden, das solche Zusammensetzungen und Nahrungsergänzungsmittel, insbesondere in homogener Durchmischung, ermöglicht.

Erfindungsgemäß wird diese Aufgabe durch eine Zusammensetzung nach Anspruch 1 gelöst. Mit Blick auf die Zusammensetzung als Nahrungsergänzungsmittel für Kinder wird diese Aufgabe durch Anspruch 3 gelöst. Mit Blick auf das Verfahren zur Herstellung einer Zusammensetzung, eines Nahrungsergänzungsmittels oder einer Zusammensetzung als Nahrungsergänzungsmittel für Kinder wird diese Aufgabe durch Anspruch 4 gelöst.

Die Erfindung beruht auf dem Gedanken, eine Zusammensetzung, enthaltend eine Pfefferkomponente, eine Curcumin-Cyclodextrin-Mischung und eine Mischung aus Früchten, Gemüse und Gewürzen, anzugeben.

Die erfindungsgemäße Mischung aus Früchten, Gemüse und Gewürzen enthält vorzugsweise Konzentrate und Extrakte verschiedenster Pflanzen und stellt eine Vielzahl gesundheitsfördernder pflanzlicher Sekundärmetabolite, Vitamine und Polyphenole bereit.

Eine erfindungsgemäße Zusammensetzung kann die Bioverfügbarkeit der in der Zusammensetzung enthaltenen Verbindungen erhöhen. Daher ergibt sich eine Zusammensetzung, die eine besonders gesundheitsfördernde Wirkung hat und eine ausgewogene Ernährung besonders gut unterstützt.

Ein weiterer Vorteil besteht darin, dass die Zusammensetzung verschiedenen Nahrungsergänzungsmitteln mit geringem Aufwand beigemischt werden kann, wodurch verschiedene Nahrungsergänzungsmittel von den Eigenschaften der Zusammensetzung profitieren.

Bevorzugte Ausführungsformen der Erfindung sind in den Unteransprüchen angegeben.

Die Zusammensetzung enthält ferner eine Algenkomponente.

Die Algenkomponente stammt aus Braunalgen.

Die Algenkomponente ist ein Algenpulver bzw. ein Pulver aus Algen, insbesondere aus getrockneten Algen. Ein Pulver aus Algen wird insbesondere durch Vermahlen von Algen, insbesondere durch Vermahlen von getrockneten Algen, erhalten.

Die Algenkomponente stammt aus Braunalgen und ist ein Pulver aus Braunalgen. Algen enthalten wertvolle und natürliche organische Substanzen und Mineralien.

Die Zusammensetzung enthält ferner einen Extrakt aus dem Ingwer (Zingiber officinale). Bevorzugt handelt es sich um einen Extrakt aus dem Rhizom des Ingwers (Zingiber officinale).

Ein Ingwerextrakt enthält wertvolle und natürliche organische Substanzen. Darüber hinaus ist ein Ingwerextrakt ein Bioenhancer, der die Bioverfügbarkeit von Komponenten der Zusammensetzung steigert.

Die Pfefferkomponente ist Piperin.

Durch Piperin wird die Bioverfügbarkeit der Komponenten der Zusammensetzung gesteigert.

Die Curcumin-Cyclodextrin-Mischung ist ein Curcumin-Gamma-Cyclodextrin-Komplex. Bei einer derartigen Ausbildung kann Curcumin besonders effektiv aufgenommen werden und wirken.

In bevorzugten Ausführungsformen enthält die Mischung aus Früchten, Gemüse und Gewürzen
einen Extrakt aus grünem Tee (Camellia sinensis), insbesondere aus Blättern des grünen Tees, und
ein Konzentrat aus dem Broccoli (Brassica oleracea italica), insbesondere aus Samen, Keimen und/oder Sprossen des Broccolis, und
ein Konzentrat aus dem Broccoli (Brassica oleracea italica), insbesondere aus Röschen und/oder Stielen des Broccolis, und
einen Extrakt aus der Zwiebel (Allium cepa, insbesondere Allium cepa alliaceae), insbesondere aus der Zwiebel der Zwiebel, und
einen Extrakt aus dem Apfel (Malus domestica), insbesondere aus der Apfelfrucht, insbesondere aus Schalen der Apfelfrucht, und
einen Extrakt aus der Acerola (Malpighia glabra, insbesondere Malpighia glabra linne), insbesondere aus der Frucht der Acerola, und
ein Konzentrat aus der Tomate (Lycopersicon esculentum), insbesondere aus der Frucht der Tomate, und
ein Konzentrat aus der Kurkuma (Curcuma longa), insbesondere aus der Wurzel der Kurkuma, und
ein Konzentrat aus dem Knoblauch (Allium sativum), insbesondere aus der Knoblauchzehe, und
ein Konzentrat aus dem Basilikum (Ocimum basilicum), insbesondere aus Blättern des Basilikums, und
ein Konzentrat aus dem Oregano (Origanum vulgare), insbesondere aus Blättern des Oreganos, und
ein Konzentrat aus dem Zimtbaum (Cinnamonum cassia), insbesondere aus der Rinde (ohne Kork) des Zimtbaumes, und
ein Konzentrat aus der Karotte (Dacus carota, insbesondere Dacus carota sativa), insbesondere aus der Wurzel der Karotte, und
ein Konzentrat aus dem Holunder (Sambucus nigra), insbesondere aus der Frucht des Holunders, und
einen Extrakt aus der Johannisbeere (Ribes), insbesondere der schwarzen Johannisbeere (Ribes nigrum), insbesondere aus der Frucht der schwarzen Johannisbeere, und
ein Konzentrat aus der Heidelbeere (Vaccinium), insbesondere aus der Frucht der Heidelbeere, und
ein Konzentrat aus der Himbeere (Rubus idaeus), insbesondere aus der Frucht der Himbeere, und
ein Konzentrat aus der Brombeere (Rubus spp.), insbesondere aus der Frucht der Brombeere, und
ein Konzentrat aus der Apfelbeere (Aronia, insbesondere Aronia melanocarpa), insbesondere aus der Frucht der Apfelbeere, und
ein Konzentrat aus dem Spinat (Spinacia oleracea), insbesondere aus den Blättern des Spinates, und
ein Konzentrat aus der Kirsche (Prunus avium), insbesondere aus der Kirschfrucht, und
ein Konzentrat aus dem Rosenkohl (Brassica oleracea gemmifera), insbesondere aus Röschen des Rosenkohles.

In bestimmten Ausführungsformen enthält die Mischung aus Früchten, Gemüse und Gewürzen
einen Extrakt aus grünem Tee (Camellia sinensis), insbesondere aus Blättern des grünen Tees, und/oder
ein Konzentrat aus dem Broccoli (Brassica oleracea italica), insbesondere aus Samen, Keimen und/oder Sprossen des Broccolis, und/oder
ein Konzentrat aus dem Broccoli (Brassica oleracea italica), insbesondere aus Röschen und/oder Stielen des Broccolis, und/oder
einen Extrakt aus der Zwiebel (Allium cepa, insbesondere Allium cepa alliaceae), insbesondere aus der Zwiebel der Zwiebel, und/oder
einen Extrakt aus dem Apfel (Malus domestica), insbesondere aus der Apfelfrucht, insbesondere aus Schalen der Apfelfrucht, und/oder
einen Extrakt aus der Acerola (Malpighia glabra, insbesondere Malpighia glabra linne), insbesondere aus der Frucht der Acerola, und/oder
ein Konzentrat aus der Tomate (Lycopersicon esculentum), insbesondere aus der Frucht der Tomate, und/oder
ein Konzentrat aus der Kurkuma (Curcuma longa), insbesondere aus der Wurzel der Kurkuma, und/oder
ein Konzentrat aus dem Knoblauch (Allium sativum), insbesondere aus der Knoblauchzehe, und/oder
ein Konzentrat aus dem Basilikum (Ocimum basilicum), insbesondere aus Blättern des Basilikums, und/oder
ein Konzentrat aus dem Oregano (Origanum vulgare), insbesondere aus Blättern des Oreganos, und/oder
ein Konzentrat aus dem Zimtbaum (Cinnamonum cassia), insbesondere aus der Rinde (ohne Kork) des Zimtbaumes, und/oder
ein Konzentrat aus der Karotte (Dacus carota, insbesondere Dacus carota sativa), insbesondere aus der Wurzel der Karotte, und/oder
ein Konzentrat aus dem Holunder (Sambucus nigra), insbesondere aus der Frucht des Holunders, und/oder
einen Extrakt aus der Johannisbeere (Ribes), insbesondere der schwarzen Johannisbeere (Ribes nigrum), insbesondere aus der Frucht der schwarzen Johannisbeere, und/oder
ein Konzentrat aus der Heidelbeere (Vaccinium), insbesondere aus der Frucht der Heidelbeere, und/oder
ein Konzentrat aus der Himbeere (Rubus idaeus), insbesondere aus der Frucht der Himbeere, und/oder
ein Konzentrat aus der Brombeere (Rubus spp.), insbesondere aus der Frucht der Brombeere, und/oder
ein Konzentrat aus der Apfelbeere (Aronia, insbesondere Aronia melanocarpa), insbesondere aus der Frucht der Apfelbeere, und/oder
ein Konzentrat aus dem Spinat (Spinacia oleracea), insbesondere aus den Blättern des Spinates, und/oder
ein Konzentrat aus der Kirsche (Prunus avium), insbesondere aus der Kirschfrucht, und/oder
ein Konzentrat aus dem Rosenkohl (Brassica oleracea gemmifera), insbesondere aus Röschen des Rosenkohles.

Der Begriff "Extrakt" (bevorzugt mit Ausnahme des Algenextraktes) bezeichnet im Rahmen dieser Patentanmeldung einen eingetrockneten Auszug aus entsprechenden Pflanzenteilen, wobei der Extrakt bevorzugt mit Hilfe von Ethanol und Wasser und mit einem Droge-Extrakt-Verhältnis zwischen 90:1 und 15:1 gewonnen wird. Ein "Konzentrat" bezeichnet im Rahmen dieser Patentanmeldung ein entwässertes Produkt aus entsprechenden Pflanzenteilen. Extrakte und Konzentrate aus Früchten, Blättern, Zwiebeln, Rinde (ohne Kork), Wurzeln, Rhizomen, Knollen, Samen, Keimen, Sprossen, Röschen und Stielen bieten je nach Pflanzenart einen hohen Gehalt an wertvollen, pflanzlichen Sekundärmetaboliten wie Polyphenolen. Grundsätzlich liegt ein Gedanke darin, mehrere Extrakte und Konzentrate aus verschieden Pflanzenarten und -teilen bereitzustellen, um einen möglichst hohen Gehalt und vor allem eine möglichst hohe Vielfalt pflanzlicher Sekundärmetabolite und Polyphenole bereitzustellen. Daher ist eine große Anzahl an Extrakten und Konzentraten aus verschiedenen Pflanzenteilen verschiedener Pflanzenarten bevorzugt. Polyphenole sind aromatische Verbindungen, die zwei oder mehr direkt an einen aromatischen Ring gebundene Hydroxylgruppen enthalten und zu den pflanzlichen Sekundärmetaboliten gerechnet werden. Polyphenole weisen verschiedene gesundheitsfördernde Effekte auf, insbesondere wirken einige Polyphenole antioxidativ.

100 g der Zusammensetzung enthalten 10 mg bis 5000 mg, bevorzugt 50 mg bis 4000 mg, der Pfefferkomponente, 20 mg bis 1000 mg, bevorzugt 80 mg bis 400 mg, der Curcumin-Cyclodextrin-Mischung, und 50 mg bis 1000 mg, bevorzugt 100 mg bis 500 mg, der Mischung aus Früchten, Gemüse und Gewürzen. Darüber hinaus enthalten 100 g der Zusammensetzung 1 mg bis 1000 mg, bevorzugt 5 mg bis 60 mg, des Extraktes aus dem Ingwer (Zingiber officinale) und 5 mg bis 500 mg, bevorzugt 10 mg bis 100 mg, der Algenkomponente.

Die Zusammensetzung enthält ferner:
- Vitamin C, 500 mg bis 2000 mg pro 100 g Zusammensetzung;
- Niacin, 100 mg bis 800 mg pro 100 g Zusammensetzung;
- Koffein, 50 mg bis 500 mg pro 100 g Zusammensetzung;
- Alpha-Tocopherylacetat, 20 mg bis 300 mg pro 100 g Zusammensetzung;
- Pantothensäure, 20 mg bis 100 mg pro 100 g Zusammensetzung;
- Pyridoxin, 5 mg bis 60 mg pro 100 g Zusammensetzung;
- Thiamin, 5 mg bis 60 mg pro 100 g Zusammensetzung;
- Riboflavin, 4 mg bis 40 mg pro 100 g Zusammensetzung;
- Cyanocobolamin, 4 µg bis 20 µg pro 100 g Zusammensetzung;
- Folsäure, 0,5 mg bis 5 mg pro 100 g Zusammensetzung;
- Biotin, 0,5 mg bis 5 mg pro 100 g Zusammensetzung;
- Beta-Carotin, 4 mg bis 30 mg pro 100 g Zusammensetzung;
- Mit Selen angereicherte Hefe, 10 mg bis 500 mg pro 100 g Zusammensetzung
- Gummi arabicum, 10 g bis 30 g pro 100 g Zusammensetzung;
- Pflanzliche Fasern, aus Hafer, Erbse, Reis und Apfel, 10 g bis 25 g pro 100 g Zusammensetzung;
- Pectin und/oder Guargummi, insgesamt 1 g bis 6 g;
- Guaranaextrakt, 1 g bis 3 g pro 100 g Zusammensetzung;
- Milchsäurekulturen (Lactobacillus acidophilus, Lactobacillus reuteri) 0,1 g bis 1 g pro 100 g Zusammensetzung;
- Inulin, 0,1 g bis 1 g pro 100 g Zusammensetzung;
- Traubenkernextrakt, 1 mg bis 20 mg pro 100 g Zusammensetzung.

Ferner enthält die Zusammensetzung:
- Citronensäure, 1 g bis 5 g pro 100 g Zusammensetzung;
- Fructose, 20 g bis 70 g pro 100 g Zusammensetzung;
- Rote-Bete-Pulver (Konzentrat des Saftes roter Bete, Maltodextrin und Citronensäure), 0,1 g bis 2 g pro 100 g Zusammensetzung;
- Multienzymkomplex aus Amylase, Lactase, Protease, Cellulase, Lipase, 0,1 g bis 1 g pro 100 g Zusammensetzung;
- Steviolglykoside, 0,01 g bis 0,5 g pro 100 g Zusammensetzung.

In bevorzugten Ausführungsformen liegt die Zusammensetzung als Pulver vor. In bevorzugten Ausführungsformen liegt die Zusammensetzung als Pulver in Kapseln, Beuteln oder Dosen, insbesondere in Beuteln, vor. Ganz besonders bevorzugt liegt die Zusammensetzung als Pulver in Einzeldosen in Beuteln vor.

In bestimmten Ausführungsformen eignet sich die Zusammensetzung zur Herstellung einer wässrigen Lösung in Wasser. In bestimmten Ausführungsformen eignet sich die Zusammensetzung zur Herstellung einer Suspension in Wasser.

Die Erfindung beruht ferner auf dem Gedanken, eine Zusammensetzung als Nahrungsergänzungsmittel für Kinder anzugeben.

Die Erfindung beruht auf dem Gedanken, eine Zusammensetzung als Nahrungsergänzungsmittel für Kinder anzugeben, wobei die Zusammensetzung eine Pfefferkomponente, eine Curcumin-Cyclodextrin-Mischung und eine Mischung aus Früchten, Gemüse und Gewürzen enthält.

Mit anderen Worten soll eine Zusammensetzung bereitgestellt werden, die ähnliche Wirkungen und Vorteile aufweist wie die zuvor beschriebene Zusammensetzung, allerdings in ihrer Zusammensetzung speziell auf die Bedürfnisse und Empfindlichkeiten von Kindern angelegt ist. Bisherige Erklärungen und Beschreibungen zu der erfindungsgemäßen Zusammensetzung und deren Vorteilen treffen daher auch auf die Zusammensetzung als Nahrungsergänzungsmittel für Kinder zu, sofern sich aus dem Nachfolgenden nichts Gegenteiliges ergibt.

Die Pfefferkomponente ist ein Extrakt aus dem Pfeffer (Piper nigrum), nämlich Piperin.

Durch Piperin wird die Bioverfügbarkeit der Komponenten eines Nahrungsergänzungsmittels gesteigert.

Die Curcumin-Cyclodextrin-Mischung ist ein Curcumin-Gamma-Cyclodextrin-Komplex. Solche Curcumin-Gamma-Cyclodextrin-Komplexe sind kommerziell erhältlich.

Bei einer derartigen Ausbildung kann Curcumin besonders effektiv aufgenommen werden und wirken.

In bevorzugten Ausführungsformen enthält die Mischung aus Früchten, Gemüse und Gewürzen
einen Extrakt aus grünem Tee (Camellia sinensis), insbesondere aus Blättern des grünen Tees, und
ein Konzentrat aus dem Broccoli (Brassica oleracea italica), insbesondere aus Samen, Keimen und/oder Sprossen des Broccolis, und
ein Konzentrat aus dem Broccoli (Brassica oleracea italica), insbesondere aus Röschen und/oder Stielen des Broccolis, und
einen Extrakt aus der Zwiebel (Allium cepa, insbesondere Allium cepa alliaceae), insbesondere aus der Zwiebel der Zwiebel, und
einen Extrakt aus dem Apfel (Malus domestica), insbesondere aus der Apfelfrucht, insbesondere aus Schalen der Apfelfrucht, und
einen Extrakt aus der Acerola (Malpighia glabra, insbesondere Malpighia glabra linne), insbesondere aus der Frucht der Acerola, und
ein Konzentrat aus der Tomate (Lycopersicon esculentum), insbesondere aus der Frucht der Tomate, und
ein Konzentrat aus der Kurkuma (Curcuma longa), insbesondere aus der Wurzel der Kurkuma, und
ein Konzentrat aus dem Knoblauch (Allium sativum), insbesondere aus der Knoblauchzehe, und
ein Konzentrat aus dem Basilikum (Ocimum basilicum), insbesondere aus Blättern des Basilikums, und
ein Konzentrat aus dem Oregano (Origanum vulgare), insbesondere aus Blättern des Oreganos, und
ein Konzentrat aus dem Zimtbaum (Cinnamonum cassia), insbesondere aus der Rinde (ohne Kork) des Zimtbaumes, und
ein Konzentrat aus der Karotte (Dacus carota, insbesondere Dacus carota sativa), insbesondere aus der Wurzel der Karotte, und
ein Konzentrat aus dem Holunder (Sambucus nigra), insbesondere aus der Frucht des Holunders, und
einen Extrakt aus der Johannisbeere (Ribes), insbesondere der schwarzen Johannisbeere (Ribes nigrum), insbesondere aus der Frucht der schwarzen Johannisbeere, und
ein Konzentrat aus der Heidelbeere (Vaccinium), insbesondere aus der Frucht der Heidelbeere, und
ein Konzentrat aus der Himbeere (Rubus idaeus), insbesondere aus der Frucht der Himbeere, und
ein Konzentrat aus der Brombeere (Rubus spp.), insbesondere aus der Frucht der Brombeere, und
ein Konzentrat aus der Apfelbeere (Aronia, insbesondere Aronia melanocarpa), insbesondere aus der Frucht der Apfelbeere, und
ein Konzentrat aus dem Spinat (Spinacia oleracea), insbesondere aus den Blättern des Spinates, und
ein Konzentrat aus der Kirsche (Prunus avium), insbesondere aus der Kirschfrucht, und
ein Konzentrat aus dem Rosenkohl (Brassica oleracea gemmifera), insbesondere aus Röschen des Rosenkohles.

In bestimmten Ausführungsformen enthält die Mischung aus Früchten, Gemüse und Gewürzen
einen Extrakt aus grünem Tee (Camellia sinensis), insbesondere aus Blättern des grünen Tees, und/oder
ein Konzentrat aus dem Broccoli (Brassica oleracea italica), insbesondere aus Samen, Keimen und/oder Sprossen des Broccolis, und/oder
ein Konzentrat aus dem Broccoli (Brassica oleracea italica), insbesondere aus Röschen und/oder Stielen des Broccolis, und/oder
einen Extrakt aus der Zwiebel (Allium cepa, insbesondere Allium cepa alliaceae), insbesondere aus der Zwiebel der Zwiebel, und/oder
einen Extrakt aus dem Apfel (Malus domestica), insbesondere aus der Apfelfrucht, insbesondere aus Schalen der Apfelfrucht, und/oder
einen Extrakt aus der Acerola (Malpighia glabra, insbesondere Malpighia glabra linne), insbesondere aus der Frucht der Acerola, und/oder
ein Konzentrat aus der Tomate (Lycopersicon esculentum), insbesondere aus der Frucht der Tomate, und/oder
ein Konzentrat aus der Kurkuma (Curcuma longa), insbesondere aus der Wurzel der Kurkuma, und/oder
ein Konzentrat aus dem Knoblauch (Allium sativum), insbesondere aus der Knoblauchzehe, und/oder
ein Konzentrat aus dem Basilikum (Ocimum basilicum), insbesondere aus Blättern des Basilikums, und/oder
ein Konzentrat aus dem Oregano (Origanum vulgare), insbesondere aus Blättern des Oreganos, und/oder
ein Konzentrat aus dem Zimtbaum (Cinnamonum cassia), insbesondere aus der Rinde (ohne Kork) des Zimtbaumes, und/oder
ein Konzentrat aus der Karotte (Dacus carota, insbesondere Dacus carota sativa), insbesondere aus der Wurzel der Karotte, und/oder
ein Konzentrat aus dem Holunder (Sambucus nigra), insbesondere aus der Frucht des Holunders, und/oder
einen Extrakt aus der Johannisbeere (Ribes), insbesondere der schwarzen Johannisbeere (Ribes nigrum), insbesondere aus der Frucht der schwarzen Johannisbeere, und/oder
ein Konzentrat aus der Heidelbeere (Vaccinium), insbesondere aus der Frucht der Heidelbeere, und/oder
ein Konzentrat aus der Himbeere (Rubus idaeus), insbesondere aus der Frucht der Himbeere, und/oder
ein Konzentrat aus der Brombeere (Rubus spp.), insbesondere aus der Frucht der Brombeere, und/oder
ein Konzentrat aus der Apfelbeere (Aronia, insbesondere Aronia melanocarpa), insbesondere aus der Frucht der Apfelbeere, und/oder
ein Konzentrat aus dem Spinat (Spinacia oleracea), insbesondere aus den Blättern des Spinates, und/oder
ein Konzentrat aus der Kirsche (Prunus avium), insbesondere aus der Kirschfrucht, und/oder
ein Konzentrat aus dem Rosenkohl (Brassica oleracea gemmifera), insbesondere aus Röschen des Rosenkohles.

100 g der Zusammensetzung als Nahrungsergänzungsmittel für Kinder enthalten
- 50 mg bis 4000 mg der Pfefferkomponente,
- 8 mg bis 60 mg der Curcumin-Cyclodextrin-Mischung und
- 100 mg bis 500 mg der Mischung aus Früchten, Gemüse und Gewürzen.

Ferner enthält die Zusammensetzung als Nahrungsergänzungsmittel für Kinder:
- Vitamin C, 500 mg bis 2000 mg pro 100 g;
- Niacin, 50 mg bis 600 mg pro 100 g;
- Alpha-Tocopherylacetat, 80 mg bis 300 mg pro 100 g;
- Pantothensäure, 50 mg bis 100 mg pro 100 g;
- Pyridoxin, 5 mg bis 40 mg pro 100 g;
- Thiamin, 5 mg bis 50 mg pro 100 g;
- Riboflavin, 4 mg bis 40 mg pro 100 g;
- Cyanocobolamin, 10 µg bis 50 µg pro 100 g;
- Folsäure, 0,5 mg bis 3 mg pro 100 g;
- Biotin, 0,2 mg bis 1 mg pro 100 g;
- Beta-Carotin, 10 mg bis 50 mg pro 100 g;
- Chrompicolinat und/oder Chromtrichlorid, jeweils 0,5 mg bis 5 mg pro 100 g;
- Mit Selen angereicherte Hefe, 50 bis 400 mg pro 100 g;
- Zinkgluconat, 100 mg bis 500 mg pro 100 g;
- Kupfer(II)gluconat, 20 mg bis 80 mg pro 100 g;
- Cholecalciferol, 50 µg bis 250 µg pro 100 g;
- Trimagnesiumcitrat, 3 g bis 11 g pro 100 g;
- Calciumhydrogenphosphat, 0,5 g bis 3 g pro 100 g;
- Caciumlactat, 0,2 g bis 1,0 g pro 100 g;
- Milchmineralkonzentrat, 3 g bis 15 g pro 100 g;
- Inulin, 40 g bis 80 g pro 100 g.

Ferner enthält die Zusammensetzung als Nahrungsergänzungsmittel für Kinder:
- Citronensäure, 3 g bis 11 g pro 100 g;
- Steviolglykoside, 0,01 g bis 1,0 g pro 100 g.

In bevorzugten Ausführungsformen enthält die Zusammensetzung als Nahrungsergänzungsmittel für Kinder keine Algenkomponente und keinen Extrakt aus dem Ingwer.

Eine weitere Aufgabe der Erfindung ist es, ein Verfahren zur Herstellung einer Zusammensetzung oder einer Zusammensetzung als Nahrungsergänzungsmittel für Kinder anzugeben. Das Verfahren umfasst:
- Das Bereitstellen in einem Gefäß und Rühren
   oder Pfefferkomponente,
   oder Curcumin-Cyclodextrin-Mischung,
   ∘ optional des Extraktes aus dem Ingwer (Zingiber officinale),
   ∘ optional der Algenkomponente und
   ∘ der Mischung aus Früchten, Gemüse und Gewürzen;
- Das Zugeben weiterer Komponenten und Rühren, um ein Zwischenprodukt zu erhalten,
   wobei das Zugaben mindestens einer Komponente bevorzugt a) portionsweise in Teilmengen einer Gesamtmenge der Komponente und unterbrochen durch Rühren oder b) kontinuierlich unter Rühren erfolgt;
- Abfüllen des Zwischenproduktes in Dosen, Beutel oder Kapseln.

Das Bereitstellen und Zugeben erfolgt bevorzugt derart, dass die Pfefferkomponente in 10 mg bis 5000 mg, bevorzugt 50 mg bis 4000 mg, pro 100 g Zwischenprodukt vorliegt.

Das Bereitstellen und Zugeben erfolgt bevorzugt derart, dass die Mischung aus Früchten, Gemüse und Gewürzen in 50 mg bis 1000 mg, bevorzugt 100 mg bis 500 mg, pro 100 g Zwischenprodukt vorliegt.

Optional erfolgt das Bereitstellen und Zugeben derart, dass das Zwischenprodukt pro 100 g 1 mg bis 1000 mg, bevorzugt 5 mg bis 60 mg, des Extraktes aus dem Ingwer (Zingiber officinale) enthält.

Optional erfolgt das Bereitstellen und Zugeben derart, dass das Zwischenprodukt pro 100 g 5 mg bis 500 mg, bevorzugt 10 mg bis 100 mg, der Algenkomponente enthält.

Bei der Herstellung einer Zusammensetzung oder eines Nahrungsergänzungsmittels erfolgt das Bereitstellen und Zugeben bevorzugt derart, dass die Curcumin-Cyclodextrin-Mischung in 20 mg bis 1000 mg, besonders bevorzugt 80 mg bis 400 mg, pro 100 g Zwischenprodukt vorliegt. Bei der Herstellung einer Zusammensetzung als Nahrungsergänzungsmittel für Kinder erfolgt das Bereitstellen und Zugeben bevorzugt derart, dass die Curcumin-Cyclodextrin-Mischung in 8 mg bis 60 mg pro 100 g Zwischenprodukt vorliegt.

Für die Zusammensetzung als Nahrungsergänzungsmittel für Kinder sind die Algenkomponente und der Extrakt aus dem Ingwer nicht vorgesehen.

Das Zugaben mindestens einer Komponente erfolgt bevorzugt portionsweise in Teilmengen einer Gesamtmenge der Komponente und unterbrochen durch Rühren oder kontinuierlich unter Rühren. Mit anderen Worten wird mindestens eine Komponente nicht auf einmal zugegeben, sondern derart, dass sich dieser nach und nach mit den anderen Komponenten vermischen kann. Dies ist insbesondere für Komponenten mit großem Gewichtsanteil, z.B. Fructose oder Inulin, relevant. Hierdurch kann ein effizientes Vermischen und ein homogenes Produkt gewährleistet werden.

Die Erfindung wird nachfolgend anhand eines Ausführungsbeispiels näher erläutert.

In einem erfindungsgemäßen Beispiel der Zusammensetzung enthalten 100 g der Zusammensetzung 1000 mg Piperin, 130 mg Curcumin-Gamma-Cyclodextrin-Komplex, 20 mg eines Extraktes aus dem Ingwer (Zingiber officinale), 33 mg eines Pulvers aus Braunalgen und 250 mg einer Mischung aus Früchten, Gemüse und Gewürzen.

Die Mischung aus Früchten, Gemüse und Gewürzen enthält einen Extrakt aus Blättern des grünen Tees (Camellia sinensis), ein Konzentrat aus Broccolisprossen (Brassica oleracea italica), einen Extrakt aus der Zwiebel der Zwiebel (Allium cepa), einen Extrakt aus Schalen der Apfelfrucht (Malus domestica), einen Extrakt aus der Frucht der Acerola (Malpighia glabra), ein Konzentrat aus der Frucht der Tomate (Lycopersicon esculentum), ein Konzentrat aus Röschen und/oder Stielen des Broccolis (Brassica oleracea italica), ein Konzentrat aus der Wurzel der Kurkuma (Curcuma longa), ein Konzentrat aus der Zehe von Knoblauch (Allium sativum), ein Konzentrat aus Basilikumblättern (Ocimum basilicum), ein Konzentrat aus Oreganoblättern (Origanum vulgare), ein Konzentrat aus der Rinde (ohne Kork) des Zimtbaumes (Cinnamonum cassia), ein Konzentrat aus der Wurzel der Karotte (Dacus carota), ein Konzentrat aus der Frucht des Holunders (Sambucus nigra), einen Extrakt aus der Frucht der Johannisbeere (Ribes), ein Konzentrat aus der Frucht der Heidelbeere (Vaccinium), ein Konzentrat aus der Frucht der Himbeere (Rubus idaeus), ein Konzentrat aus der Frucht Brombeere (Rubus spp.), ein Konzentrat aus Frucht der Apfelbeere (Aronia), ein Konzentrat aus Spinatblättern (Spinacia oleracea), ein Konzentrat aus der Kirschfrucht (Prunus avium), einen Extrakt aus der Frucht der Heidelbeere (Vaccinium) und ein Konzentrat aus Röschen des Rosenkohles (Brassica oleracea gemmifera).

Zu großem Anteil enthält die Mischung aus Früchten, Gemüse und Gewürzen einen Extrakt aus Blättern des grünen Tees (ca. 30 Gew.-%), ein Konzentrat aus Sprossen des Broccolis (ca. 10 Gew.-%), einen Extrakt aus der Zwiebel der Zwiebel (ca. 9 Gew.%), einen Extrakt aus Schalen der Apfelfrucht (ca. 9 Gew.-%), einen Extrakt aus der Frucht der Acerola (ca. 4 Gew.-%), ein Konzentrat aus der Frucht der Tomate (ca. 4 Gew.-%) und ein Konzentrat (ca. 4 Gew.-%) aus Röschen und/oder Stielen des Broccolis. Die übrigen Bestandteile liegen in 0,1 Gew.-% bis 3,5 Gew.-% vor.

Mit der erfindungsgemäßen Zusammensetzung können außerdem verschiedene andere Zusammensetzungen und Nahrungsergänzungsmittel hergestellt werden, welche alle erheblich von den gesundheitsfördernden und resorptionssteigernden Eigenschaften der erfindungsgemäßen Zusammensetzung profitieren.

So enthalten 100 g der Zusammensetzung dieses Beispiels ferner:
- Vitamin C, 1000 mg;
- Niacin, 340 mg;
- Koffein, 200 mg;
- Alpha-Tocopherylacetat, 20 mg;
- Pantothensäure, 60 mg;
- Pyridoxin, 20 mg;
- Thiamin, 14 mg;
- Riboflavin, 16 mg;
- Cyanocobolamin, bevorzugt 10 µg;
- Folsäure, 2,0 mg;
- Biotin, 1,5 mg;
- Beta-Carotin, 13 mg;
- Mit Selen angereicherte Hefe, 91 mg;
- Citronensäure, 2,6 g;
- Gummi arabicum, 20 g;
- Pflanzliche Fasern aus Hafer, Erbse, Reis und Apfel, 17 g;
- Pectin, 1 g;
- Guargummi, 3 g;
- Guaranaextrakt, 3 g;
- Rote-Bete-Pulver (Konzentrat des Saftes roter Bete, Maltodextrin und Citronensäure), 0,7 g;
- Milchsäurekulturen (Lactobacillus acidophilus, Lactobacillus reuteri), 0,3 g;
- Multienzymkomplex aus Amylase, Lactase, Protease, Cellulase, Lipase, 0,3 g;
- Inulin, 0,3 g;
- Steviolglykoside, 0,1 g;
- Traubenkernextrakt 6 mg;
- Fructose, ad 100 g.

Dieses Beispiel beschreibt auch ein Nahrungsergänzungsmittel, das diese Zusammensetzung enthält. In diesem Ausführungsbeispiel liegt die Zusammensetzung als Nahrungsergänzungsmittel in Form eines Pulvers in Beuteln vor.

In einem erfindungsgemäßen Beispiel der Zusammensetzung als Nahrungsergänzungsmittel für Kinder enthalten 100 g der Zusammensetzung als Nahrungsergänzungsmittel für Kinder 1000 mg Piperin, 14 mg Curcumin-Gamma-Cyclodextrin-Komplex, und 270 mg einer Mischung aus Früchten, Gemüse und Gewürzen.

Die Mischung aus Früchten, Gemüse und Gewürzen enthält einen Extrakt aus Blättern des grünen Tees (Camellia sinensis), ein Konzentrat aus Broccolisprossen (Brassica oleracea italica), einen Extrakt aus der Zwiebel der Zwiebel (Allium cepa), einen Extrakt aus Schalen der Apfelfrucht (Malus domestica), einen Extrakt aus der Frucht der Acerola (Malpighia glabra), ein Konzentrat aus der Frucht der Tomate (Lycopersicon esculentum), ein Konzentrat aus Röschen und/oder Stielen des Broccolis (Brassica oleracea italica), ein Konzentrat aus der Wurzel der Kurkuma (Curcuma longa), ein Konzentrat aus der Zehe von Knoblauch (Allium sativum), ein Konzentrat aus Basilikumblättern (Ocimum basilicum), ein Konzentrat aus Oreganoblättern (Origanum vulgare), ein Konzentrat aus der Rinde (ohne Kork) des Zimtbaumes (Cinnamonum cassia), ein Konzentrat aus der Wurzel der Karotte (Dacus carota), ein Konzentrat aus der Frucht des Holunders (Sambucus nigra), einen Extrakt aus der Frucht der Johannisbeere (Ribes), ein Konzentrat aus der Frucht der Heidelbeere (Vaccinium), ein Konzentrat aus der Frucht der Himbeere (Rubus idaeus), ein Konzentrat aus der Frucht Brombeere (Rubus spp.), ein Konzentrat aus Frucht der Apfelbeere (Aronia), ein Konzentrat aus Spinatblättern (Spinacia oleracea), ein Konzentrat aus der Kirschfrucht (Prunus avium), einen Extrakt aus der Frucht der Heidelbeere (Vaccinium) und ein Konzentrat aus Röschen des Rosenkohles (Brassica oleracea gemmifera).

Größtenteils enthält die Mischung aus Früchten, Gemüse und Gewürzen einen Extrakt aus Blättern des grünen Tees (ca. 30 Gew.-%), ein Konzentrat aus Sprossen des Broccolis (ca. 10 Gew.-%), ein Extrakt aus der Zwiebel der Zwiebel (ca. 9 Gew.-%), ein Extrakt aus Schalen der Apfelfrucht (ca. 9 Gew.-%), ein Extrakt aus der Frucht der Acerola (ca. 4 Gew.-%), ein Konzentrat aus der Frucht der Tomate (ca. 4 Gew.%) und ein Konzentrat (ca. 4 Gew.-%) aus Röschen und/oder Stielen des Broccolis. Die übrigen Bestandteile liegen in 0,8 Gew.-% bis 3,0 Gew.-% vor. Nur ein Konzentrat aus Röschen des Rosenkohles und ein Extrakt aus der Frucht der Heidelbeere wird der Mischung aus Früchten, Gemüse und Gewürzen in geringerer Mengen (0,1 Gew.% bis 0,3 Gew.-%) beigefügt.

Ferner enthalten 100 g dieser beispielhaften Zusammensetzung als Nahrungsergänzungsmittel für Kinder:
- Vitamin C, 860 mg;
- Niacin, 214 mg;
- Alpha-Tocopherylacetat, 143 mg;
- Pantothensäure, 72 mg;
- Pyridoxin, 23 mg;
- Thiamin, 20 mg;
- Riboflavin, 19 mg;
- Cyanocobolamin, 29 µg;
- Folsäure, 2 mg;
- Biotin, 0,4 mg;
- Beta-Carotin, 29 mg;
- Chrompicolinat, 4 mg;
- Mit Selen angereicherte Hefe, 120 mg;
- Zinkgluconat, 265 mg;
- Kupfer(II)gluconat, 46 mg;
- Cholecalciferol, 107 µg
- Citronensäure, 7 g;
- Trimagnesiumcitrat, 7 g;
- Calciumhydrogenphosphat, 1,3 g;
- Caciumlactat, 0,6 g;
- Steviolglykoside, 0,4;
- Milchmineralkonzentrat, 7 g;
- Inulin, ad 100 g.

Dieses Beispiel beschreibt eine erfindungsgemäße Zusammensetzung als Nahrungsergänzungsmittel für Kinder. In diesem Ausführungsbeispiel liegt die Zusammensetzung als Nahrungsergänzungsmittel in Form eines Pulvers in einer Dose vor.

Zuletzt soll beispielhaft ein erfindungsgemäßes Verfahren zur Herstellung einer erfindungsgemäßen Zusammensetzung beschrieben werden. Das Verfahren umfasst:
- Das Bereitstellen in einem Gefäß und Rühren von
   ∘ 1000 mg Extrakt aus dem Pfeffer,
   ∘ 130 mg der Curcumin-Cyclodextrin-Mischung,
   ∘ 20 mg des Extraktes aus dem Ingwer (Zingiber officinale),
   ∘ 33 mg Pulver aus Braunalgen und
   ∘ 250 mg der Mischung aus Früchten, Gemüse und Gewürzen;
- Das Zugeben von 1 g Vitamin C und 5 g Fructose als weitere Komponenten und Rühren. Nach Homogenisierung durch das Rühren werden weitere 15 g Fructose zugegeben und weitergerührt. Nach weiterer Homogenisierung durch das Rühren werden weitere 15 g Fructose zugegeben und weitergerührt. Dann wird die Differenz ad 100 g in Form von Fructose zugegeben und weiter durch Rühren homogenisiert. Das erhaltene Pulver wird in Beutel abgefüllt.

Durch das Verfahren kann ein effizientes Vermischen und ein homogenes Produkt gewährleistet werden.

## Patentansprüche

1. Zusammensetzung, enthaltend
∘ eine Pfefferkomponente,
∘ eine Curcumin-Cyclodextrin-Mischung,
∘ eine Mischung aus Früchten, Gemüse und Gewürzen und
∘ einen Extrakt aus dem Ingwer (Zingiber officinale),
wobei 100 g der Zusammensetzung
- 10 mg bis 5000 mg der Pfefferkomponente,
- 20 mg bis 1000 mg der Curcumin-Cyclodextrin-Mischung,
- 50 mg bis 1000 mg der Mischung aus Früchten, Gemüse und Gewürzen,
- 1 mg bis 1000 mg des Extraktes aus dem Ingwer (Zingiber officinale),
- 5 mg bis 500 mg eines Pulvers aus Braunalgen,
- 500 mg bis 2000 mg Vitamin C,
- 100 mg bis 800 mg Niacin,
- 50 mg bis 500 mg Koffein,
- 20 mg bis 300 mg Alpha-Tocopherylacetat,
- 20 mg bis 100 mg Pantothensäure,
- 5 mg bis 60 mg Pyridoxin,
- 5 mg bis 60 mg Thiamin,
- 4 mg bis 40 mg Riboflavin,
- 4 µg bis 20 µg Cyanocobolamin,
- 0,5 mg bis 5 mg Folsäure,
- 0,5 mg bis 5 mg Biotin,
- 4 mg bis 30 mg Beta-Carotin,
- 10 mg bis 500 mg mit Selen angereicherte Hefe,
- 10 g bis 30 g Gummi arabicum,
- 10 g bis 25 g pflanzliche Fasern aus Hafer, Erbse, Reis und Apfel,
- insgesamt 1 bis 6 g Pectin und/oder Guargummi,
- 1 g bis 3 g Guaranaextrakt,
- 0,1 g bis 1 g Lactobacillus acidophilus, Lactobacillus reuteri,
- 0,1 g bis 1 g Inulin,
- 1 mg bis 20 mg Traubenkernextrakt,
- 1 g bis 5 g Citronensäure,
- 20 g bis 70 g Fructose,
- 0,1 g bis 2 g Rote-Bete-Pulver,
- 0,1 g bis 1 g Multienzymkomplex aus Amylase, Lactase, Protease, Cellulase, Lipase,
0,01 g bis 0,5 g Steviolglykoside
enthalten,
wobei die Pfefferkomponente Piperin ist,
wobei die Curcumin-Cyclodextrin-Mischung ein Curcumin-Gamma-Cyclodextrin-Komplex ist.

2. Zusammensetzung nach Anspruch 1, wobei die Mischung aus Früchten, Gemüse und Gewürzen einen Extrakt aus grünem Tee (Camellia sinensis), ein Konzentrat aus Broccoli (Brassica oleracea italica), einen Extrakt aus der Zwiebel (Allium cepa), einen Extrakt aus dem Apfel (Malus domestica), einen Extrakt aus der Acerola (Malpighia glabra), ein Konzentrat aus der Tomate (Lycopersicon esculentum), ein Konzentrat aus der Kurkuma (Curcuma longa), ein Konzentrat aus dem Knoblauch (Allium sativum), ein Konzentrat aus dem Basilikum (Ocimum basilicum), ein Konzentrat aus dem Oregano (Origanum vulgare), ein Konzentrat aus dem Zimtbaum (Cinnamonum cassia), ein Konzentrat aus der Karotte (Dacus carota), ein Konzentrat aus dem Holunder (Sambucus nigra), einen Extrakt aus der Johannisbeere (Ribes), ein Konzentrat aus der Heidelbeere (Vaccinium), ein Konzentrat aus der Himbeere (Rubus idaeus), ein Konzentrat aus der Brombeere (Rubus spp.), ein Konzentrat aus der Apfelbeere (Aronia), ein Konzentrat aus dem Spinat (Spinacia oleracea), ein Konzentrat aus der Kirsche (Prunus avium), einen Extrakt aus der Heidelbeere (Vaccinium) und ein Konzentrat aus dem Rosenkohl (Brassica oleracea gemmifera) enthält.

3. Zusammensetzung als Nahrungsergänzungsmittel für Kinder, enthaltend
∘ eine Pfefferkomponente,
∘ eine Curcumin-Cyclodextrin-Mischung und
∘ eine Mischung aus Früchten, Gemüse und Gewürzen
wobei 100 g der Zusammensetzung
- 50 mg bis 4000 mg der Pfefferkomponente
- 8 mg bis 60 mg der Curcumin-Cyclodextrin-Mischung,
- 100 mg bis 500 mg der Mischung aus Früchten, Gemüse und Gewürzen,
- 500 mg bis 2000 mg pro 100 g Vitamin C,
- 50 mg bis 600 mg pro 100 g Niacin,
- 80 mg bis 300 mg pro 100 g Alpha-Tocopherylacetat,
- 50 mg bis 100 mg pro 100 g Pantothensäure,
- 5 mg bis 40 mg pro 100 g Pyridoxin,
- 5 mg bis 50 mg pro 100 g Thiamin,
- 4 mg bis 40 mg pro 100 g Riboflavin,
- 10 µg bis 50 µg pro 100 g Cyanocobolamin,
- 0,5 mg bis 3 mg pro 100 g Folsäure,
- 0,2 mg bis 1 mg pro 100 g Biotin,
- 10 mg bis 50 mg pro 100 g Beta-Carotin,
- Chrompicolinat und/oder Chromtrichlorid, jeweils 0,5 mg bis 5 mg,
- 50 bis 400 mg pro 100 g mit Selen angereicherte Hefe,
- 100 mg bis 500 mg pro 100 g Zinkgluconat,
- 20 mg bis 80 mg pro 100 g Kupfer(II)gluconat,
- 50 µg bis 250 µg pro 100 g Cholecalciferol,
- 3 bis 11 g pro 100 g Trimagnesiumcitrat,
- 0,5 bis 3 g pro 100 g Calciumhydrogenphosphat,
- 0,2 g bis 1,0 g pro 100 g Caciumlactat,
- 3 g bis 15 g pro 100 g Milchmineralkonzentrat,
- 40 g bis 80 g pro 100 g Inulin,
- 3 bis 11 g pro 100 g Citronensäure,
- 0,01 g bis 1,0 g pro 100 g Steviolglykoside
enthalten
wobei die Pfefferkomponente Piperin ist,
wobei die Curcumin-Cyclodextrin-Mischung ein Curcumin-Gamma-Cyclodextrin-Komplex ist.

4. Verfahren zur Herstellung einer Zusammensetzung nach einem der Ansprüche 1 bis 2 oder einer Zusammensetzung als Nahrungsergänzungsmittel für Kinder nach Anspruch 3, umfassend:
- Bereitstellen in einem Gefäß und Rühren
oder Pfefferkomponente,
oder Curcumin-Cyclodextrin-Mischung,
∘ optional des Extraktes aus dem Ingwer (Zingiber officinale),
∘ optional der Algenkomponente und
∘ der Mischung aus Früchten, Gemüse und Gewürzen;
- Zugeben weiterer Komponenten und Rühren, um ein Zwischenprodukt zu erhalten,
wobei das Zugeben mindestens einer Komponente bevorzugt a) portionsweise in Teilmengen einer Gesamtmenge der Komponente und unterbrochen durch Rühren oder b) kontinuierlich unter Rühren erfolgt;
- Abfüllen des Zwischenproduktes in Dosen, Beutel oder Kapseln.

## Claims

1. Composition, containing
∘ a pepper component,
∘ a curcumin-cyclodextrin mixture,
∘ a mixture of fruits, vegetables and spices and
∘ an extract of ginger (Zingiber officinale),
wherein 100 g of the composition contains
- 10 mg to 5000 mg of the pepper component,
- 20 mg to 1000 mg of the curcumin-cyclodextrin mixture,
- 50 mg to 1000 mg of the mixture of fruits, vegetables and spices,
- 1 mg to 1000 mg of the extract of ginger (Zingiber officinale),
- 5 mg to 500 mg of a powder from brown algae,
- 500 mg to 2000 mg of vitamin C,
- 100 mg to 800 mg of niacin,
- 50 mg to 500 mg of caffeine,
- 20 mg to 300 mg of alpha-tocopheryl acetate,
- 20 mg to 100 mg of pantothenic acid,
- 5 mg to 60 mg of pyridoxine,
- 5 mg to 60 mg of thiamine,
- 4 mg to 40 mg of riboflavin,
- 4 µg to 20 µg of cyanocobolamine,
- 0.5 mg to 5 mg of folic acid,
- 0.5 mg to 5 mg of biotin,
- 4 mg to 30 mg of beta-carotene,
- 10 mg to 500 mg of yeast enriched with selenium,
- 10 g to 30 g of gum arabic,
- 10 g to 25 g of vegetable fiber from oats, pea, rice and apple,
- a total of 1 to 6 g of pectin and/or guar gum,
- 1 g to 3 g of guarana extract,
- 0.1 g to 1 g of Lactobacillus acidophilus, Lactobacillus reuteri,
- 0.1 g to 1 g of inulin,
- 1 mg to 20 mg of grape seed extract,
- 1 g to 5 g of citric acid,
- 20 g to 70 g of fructose,
- 0.1 g to 2 g of beetroot powder,
- 0.1 g to 1 g of multienzyme complex of amylase, lactase, protease, cellulase, lipase,
0.01 g to 0.5 g of steviol glycosides,
wherein the pepper component is piperine,
wherein the curcumin-cyclodextrin mixture is a curcumin-gamma-cyclodextrin complex.

2. Composition according to claim 1, wherein the mixture of fruits, vegetables and spices contains an extract of green tea (Camellia sinensis), a concentrate of broccoli (Brassica oleracea italica), an extract of onion (Allium cepa), an extract of apple (Malus domestica), an extract of acerola (Malpighia glabra), a concentrate of tomato (Lycopersicon esculentum), a concentrate of turmeric (Curcuma longa), a concentrate of garlic (Allium sativum), a concentrate of basil (Ocimum basilicum), a concentrate of oregano (Origanum vulgare), a concentrate of cinnamon tree (Cinnamonum cassia), a concentrate of carrot (Dacus carota), a concentrate of elderberry (Sambucus nigra), an extract of currant (Ribes), a concentrate of blueberry (Vaccinium), a concentrate of raspberry (Rubus idaeus), a concentrate of blackberry (Rubus spp.), a concentrate of chokeberry (Aronia), a concentrate of spinach (Spinacia oleracea), a concentrate of cherry (Prunus avium), an extract of blueberry (Vaccinium) and a concentrate of Brussels sprout (Brassica oleracea gemmifera).

3. Composition as a food supplement for children, containing
∘ a pepper component,
∘ a curcumin-cyclodextrin mixture and
∘ a mixture of fruits, vegetables and spices,
wherein 100 g of the composition contains
- 50 mg to 4000 mg of the pepper component
- 8 mg to 60 mg of the curcumin-cyclodextrin mixture,
- 100 mg to 500 mg of the mixture of fruits, vegetables and spices,
- 500 mg to 2000 mg per 100 g vitamin C,
- 50 mg to 600 mg per 100 g niacin,
- 80 mg to 300 mg per 100 g alpha-tocopheryl acetate,
- 50 mg to 100 mg per 100 g pantothenic acid,
- 5 mg to 40 mg per 100 g pyridoxine,
- 5 mg to 50 mg per 100 g thiamine,
- 4 mg to 40 mg per 100 g riboflavin,
- 10 µg to 50 µg per 100 g cyanocobolamine,
- 0.5 mg to 3 mg per 100 g folic acid,
- 0.2 mg to 1 mg per 100 g biotin,
- 10 mg to 50 mg per 100 g beta-carotene,
- chromium picolinate and/or chromium trichloride, 0.5 mg to 5 mg each,
- 50 to 400 mg per 100 g selenium-enriched yeast,
- 100 mg to 500 mg per 100 g zinc gluconate,
- 20 mg to 80 mg per 100 g copper (II) gluconate,
- 50 µg to 250 µg per 100 g cholecalciferol,
- 3 to 11 g per 100 g trimagnesium citrate,
- 0.5 to 3 g per 100 g calcium hydrogen phosphate,
- 0.2 g to 1.0 g per 100 g calcium lactate,
- 3 g to 15 g per 100 g milk mineral concentrate,
- 40 g to 80 g per 100 g inulin,
- 3 to 11 g per 100 g citric acid,
- 0.01 g to 1.0 g per 100 g steviol glycosides
wherein the pepper component is piperine,
wherein the curcumin-cyclodextrin mixture is a curcumin-gamma-cyclodextrin complex.

4. Method for preparing a composition according to one of claims 1 to 2 or a composition as a nutritional supplement for children according to claim 3, comprising:
- providing in a vessel and stirring
∘ the pepper component,
∘ the curcumin-cyclodextrin mixture,
∘ optionally the extract from the ginger (Zingiber officinale),
∘ optionally the algae component and
∘ the mixture of fruits, vegetables and spices;
- adding further components and stirring to obtain an intermediate product,
wherein the addition of at least one component occurs preferably a) in portions in partial amounts of a total amount of the component and interrupted by stirring or b) continuously with stirring;
- filling of the intermediate product into cans, bags or capsules.

## Revendications

1. Composition, contenant
o une composante de poivre,
o un mélange de curcumine et de cyclodextrine,
o un mélange de fruits, de légumes et d'épices et
o un extrait de gingembre (*Zingiber officinale*),
dans laquelle 100 g de la composition contiennent
- 10 mg à 5000 mg de la composante de poivre,
- 20 mg à 1000 mg du mélange de curcumine et de cyclodextrine,
- 50 mg à 1000 mg du mélange de fruits, de légumes et d'épices,
- 1 mg à 1000 mg de l'extrait de gingembre (*Zingiber officinale*),
- 5 mg à 500 mg d'une poudre d'algues brunes,
- 500 mg à 2000 mg de vitamine C,
- 100 mg à 800 mg de niacine,
- 50 mg à 500 mg de caféine,
- 20 mg à 300 mg d'acétate d'alpha-tocophéryle,
- 20 mg à 100 mg d'acide pantothénique,
- 5 mg à 60 mg de pyridoxine,
- 5 mg à 60 mg de thiamine,
- 4 mg à 40 mg de riboflavine,
- 4 µg bis 20 µg de cyanocobalamine,
- 0,5 mg à 5 mg d'acide folique,
- 0,5 mg à 5 mg de biotine,
- 4 mg à 30 mg de bêtacarotène,
- 10 mg à 500 mg de levure enrichie de sélénium,
- 10 g à 30 g de gomme arabique,
- 10 g à 25 g de fibres végétales issues d'avoine, de pois, de riz et de pommes,
- 1 à 6 g au total de pectine et/ou de gomme guar,
- 1 g à 3 g d'extrait de guarana,
- 0,1 g à 1 g de *Lactobacillus acidophilus, Lactobacillus reuteri,*
- 0,1 g à 1 g d'inuline,
- 1 mg à 20 mg d'extrait de pépins de raisins,
- 1 g à 5 g d'acide citrique,
- 20 g à 70 g de fructose,
- 0,1 g à 2 g de poudre de betterave rouge,
- 0,1 g à 1 g de complexe multi-enzymes composé d'amylase, de lactase, de protéase, de cellulase, de lipase,
- 0,01 g à 0,5 g de stévioglycosides,
dans laquelle la composante de poivre est de la pipérine,
dans laquelle le mélange de curcumine et de cyclodextrine est un complexe de curcumine et de gamma-cyclodextrine.

2. Composition selon la revendication 1, dans laquelle le mélange de fruits, de légumes et d'épices contient un extrait de thé vert (*Camellia sinensis*), un concentré de brocoli (*Brassica oleracea italica*), un extrait d'oignon (*Allium cepa*), un extrait de pomme (*Malus domestica*), un extrait d'acérola (*Malpighia glabra*), un concentré de tomate (*Lycopersicon esculentum*), un concentré de curcuma (*Curcuma longa*), un concentré d'ail (*Allium sativum*), un concentré de basilic (*Ocimum basilicum*), un concentré d'origan (*Origanum vulgare*), un concentré de cannellier (*Cinnamonum cassia*), un concentré de carotte (*Daucus carota*), un concentré de sureau (*Sambucus nigra*), un extrait de cassis (*Ribes*), un concentré de myrtille (*Vaccinium*), un concentré de framboise (*Rubus idaeus*), un concentré de mûre de ronce (*Rubus* spp.), un concentré d'aronia (*Aronia*), un concentré d'épinard (*Spinacia oleracea*), un concentré de cerise (*Prunus avium*), un extrait de myrtille (*Vaccinium*) et un concentré de chou-fleur (*Brassica oleracea gemmifera*).

3. Composition utilisable comme complément alimentaire pour enfants, contenant
o une composante de poivre,
o un mélange de curcumine et de cyclodextrine et
o un mélange de fruits, de légumes et d'épices,
dans laquelle 100 g de la composition contiennent
- 50 mg à 4000 mg de la composante de poivre,
- 8 mg à 60 mg du mélange de curcumine et de cyclodextrine,
- 100 mg à 500 mg du mélange de fruits, de légumes et d'épices,
- 500 mg à 2000 mg par 100 g de vitamine C,
- 50 mg à 600 mg par 100 g de niacine,
- 80 mg à 300 mg par 100 g d'acétate d'alpha-tocophéryle,
- 50 mg à 100 mg par 100 g d'acide pantothénique,
- 5 mg à 40 mg par 100 g de pyridoxine,
- 5 mg à 50 mg par 100 g de thiamine,
- 4 mg à 40 mg par 100 g de riboflavine,
- 10 µg bis 50 µg par 100 g de cyanocobalamine,
- 0,5 mg à 3 mg par 100 g d'acide folique,
- 0,2 mg à 1 mg par 100 g de biotine,
- 10 mg à 50 mg par 100 g de bêtacarotène,
- 0,5 mg à 5 mg de picolinate de chrome et/ou 0,5 mg à 5 mg de trichlorure de chrome,
- 50 mg à 400 mg par 100 g de levure enrichie de sélénium,
- 100 mg à 500 mg par 100 g de gluconate de zinc,
- 20 mg à 80 mg par 100 g de gluconate de cuivre (II),
- 50 µg à 250 µg par 100 g de cholécalciférol,
- 3 g à 11 g par 100 g de citrate de trimagnésium,
- 0,5 g à 3 g par 100 g d'hydrogénophosphate de calcium,
- 0,2 g à 1,0 g par 100 g de lactate de calcium,
- 3 g à 15 g par 100 g de concentré de minéraux de lait,
- 40 g à 80 g par 100 g d'inuline,
- 3 g à 11 g par 100 g d'acide citrique,
- 0,01 g à 1,0 g par 100 g de stévioglycosides,
dans laquelle la composante de poivre est de la pipérine,
dans laquelle le mélange de curcumine et de cyclodextrine est un complexe de curcumine et de gamma-cyclodextrine.

4. Procédé pour la fabrication d'une composition selon l'une des revendications 1 ou 2 ou d'une composition utilisable comme complément alimentaire pour enfants selon la revendication 3, comprenant :
- la préparation dans un récipient et l'agitation
o de la composante de poivre,
o du mélange de curcumine et de cyclodextrine,
o facultativement, de l'extrait de gingembre (*Zingiber officinale*),
o facultativement, de la composante à base d'algues et
o du mélange de fruits, de légumes et d'épices ;
- ajout d'autres composantes et agitation pour obtenir un produit intermédiaire,
au moins une composante étant de préférence ajoutée a) par portions en fractions d'une quantité totale de la composante avec des interruptions par l'agitation ou b) de façon continue avec agitation ;
- conditionnement du produit intermédiaire dans des boîtes, des sachets ou des capsules.
